Europäisches Patentamt

European Patent Office

Office européen des brevets

(11) Veröffentlichungsnummer: **0 239 721**

**A2**

(12) **EUROPÄISCHE PATENTANMELDUNG**

(21) Anmeldenummer: **87100339.8**

(51) Int. Cl.⁴: **A61M 5/20**

(22) Anmeldetag: **13.01.87**

(30) Priorität: **03.04.86 DD 288724**
**01.10.86 DD 294851**

(43) Veröffentlichungstag der Anmeldung:
**07.10.87 Patentblatt 87/41**

(84) Benannte Vertragsstaaten:
**AT BE CH DE FR GB IT LI NL SE**

(71) Anmelder: **VEB Transformatoren- und Röntgenwerk "Hermann Matern" Overbeckstrasse 48 DDR-8030 Dresden(DD)**

(72) Erfinder: **von Ardenne, Manfred, Prof.Dr.h.c.mult. Zeppelinstrasse 7 DDR-8051 Dresden(DD)**
Erfinder: **Roggenbuck, Willy Sonnenleite 10 DDR-8051 Dresden(DD)**
Erfinder: **Wehnert, Jörg, Prof.Dr.sc.med. Reichenhaller Strasse 6 DDR-8051 Dresden(DD)**

(74) Vertreter: **Patentanwälte Beetz sen. - Beetz jun. Timpe - Siegfried - Schmitt-Fumian Steinsdorfstrasse 10 D-8000 München 22(DE)**

(54) **Injektionseinrichtung und Verfahren zum Einführen von Kanülen.**

(57) Die Erfindung betrifft eine Injektionseinrichtung für die Injektion von Pharmaka, z. B. für Diabetiker oder für andere spezielle Anwendungsgebiete, sowie ein Verfahren zum Einführen von Kanülen in den menschlichen oder tierischen Körper. Die Einrichtung besteht aus einem elektromotorischen Antrieb 9 für die Bewegung der Spritze 2 (Einstich) und des Kolbens 18, einer Vibrationseinrichtung 9, 10, 11, 22, die während der Bewegung der Spritze 2 auf diese eine Axialschwingung mit einer Frequenz von 40 bis 500 Hz überträgt, deren Schwingungsamplitude 0,1 bis 1,5 mm beträgt. Die Einstichtiefe der Kanüle 5 ist durch den Anschlag 16 auf 3 bis 15 mm einstellbar. Der Antrieb ist so ausgelegt, daß der gesamte Vorgang in etwa 90 s ausführbar ist. Bei Verwendung als Doppelspritze ist ein T-Stück 4 auf die Injektionsspritze 2 aufgesetzt, wobei der Abstand der Kanülen 5 vorzugsweise 7 1/2, 11 oder 14 mm beträgt.

Fig. 1

## Injektionseinrichtung und Verfahren zum Einführen von Kanülen

Die Erfindung betrifft eine Injektionseinrichtung, die aus einem Antrieb für den axialen Vorschub und die Bewegung des Kolbens einer Injektionsspritze mit mindestens einer Kanüle besteht, die auf einem beweglichen Halter auswechselbar angeordnet ist, und wobei der Antrieb Mittel zur Begrenzung der Bewegung der Injektionsspritze sowie des Kolbens aufweist, gemäß DD-PS 132 169. Sie betrifft ferner ein Verfahren zum Einführen einer oder mehrerer Kanülen in den menschlichen oder tierischen Körper.

Es sind Injektionsspritzen bekannt, die vom medizinischen Personal und auch im besonderen vom Patienten selbst benutzt werden, um ein Pharmakon zu injizieren oder auch Flüssigkeit bzw. Gewebeproben aus einem biologischen Objekt zu entnehmen.

Bekannte Injektionsspritzen (Einwegspritzen oder wiederverwendbare Spritzen) haben einen Kolben, der von Hand bewegbar ist, um durch eine Kanüle das Pharmakon aufzuziehen und durch Gegenbewegung nach Einstich in den Körper das Pharmakon zu injizieren. Bei der Injektion ist es von außerordentlicher Bedeutung, die Kanüle an der richtigen Stelle des Körpers in vorgeschriebener Richtung und Tiefe einzustechen und so dann durch Druck auf den Kolben die Spritze zu entleeren. Diese Spritzen haben bei ihrer Anwendung den Nachteil, daß der Einstich für den Patienten abhängig von der Stärke der Kanüle und vom Können des die Injektion Vornehmenden mehr oder weniger schmerzhaft ist. Hinzu kommt bei der Injektion durch den Patienten selbst (z. B. bei Diabetikern), daß vor allem bei vegetativ labilen Patienten durch eine gewisse Angst dieser Vorgang verkrampft ausgeführt wird und sich dadurch der Schmerz noch erhöht.

Ferner ist eine automatische Injektionseinrichtung bekannt, die aus einer auswechselbaren, auf einer Wiege befindlichen Injektionsspritze besteht, die Antriebsmittel besitzt, um die Kanüle und den Spritzenkolben zu bewegen. Der Kolbenhub und die Einstichtiefe werden mittels Anschlägen begrenzt. Die Spritze kann auch mit einem Mehrnadelkopf ausgerüstet sein. Zu genannten Bewegungen werden elektrische, mechanische, elektromagnetische oder pneumatische Antriebsmittel verwendet (DD-PS 132 169). Diese Injektionseinrichtung ist mit dem Mangel behaftet, daß der Einstich der Kanüle für den Patienten nach wie vor schmerzhaft ist. Schon aus diesem Grunde wäre die Injektionseinrichtung beispielsweise für den speziellen Anwendungsfall der Penis-Stimulation gänzlich ungeeignet. Ein weiterer Nachteil der Einrichtung besteht darin, daß der Einstich der Kanüle stoßartig, ausgelöst durch einen Elektromagneten, erfolgt. Dadurch wird, wiederum vor allem an empfindlichen Körperteilen, der Schmerz erhöht. Bei derartigen Injektionen kommt es auch zu Verkrampfungen und unexakten Einstichen bezüglich Tiefe und Ort.

Des weiteren ist es für das Einbringen von drahtförmigen Sonden in tiefliegende Bereiche des Körpers bekannt, daß das angespitzte Ende einer solchen Sonde mit einem Durchmesser von max. 0,2 mm mit einer Frequenz von über 1000 Hz axial schwingend mit sehr geringem Druck eingebracht wird (DD-PS 22 029). Die Schwingungen werden von einem Ultraschallschwinger erzeugt. Diese Einrichtung hat die Nachteile, daß sie einen hohen apparativen Aufwand für die Erzeugung der Schwingungen erfordert und keinen kontinuierlichen und definierten Axialvorschub ermöglicht. Weiterhin sind zur Einführung und Führung der Sonde zusätzliche Mittel auf der Hautoberfläche erforderlich, und die Einrichtung ist nicht zur Injektion von Flüssigkeiten vorgesehen bzw. verwendbar.

Der Erfindung, wie sie in den Patentansprüchen beschrieben ist, liegt die Aufgabe zugrunde, eine Injektionseinrichtung gemäß dem Gattungsbegriff des Anspruchs 1 so weiterzubilden sowie ein Verfahren zum Einführen von Kanülen in den menschlichen und tierischen Körper anzugeben, daß eine mindestens schmerzarme Injektion unter definierten Bedingungen ausführbar ist. die Injektionseinrichtung soll insbesondere für die Selbstinjektion von Pharmaka geeignet sein. Ein besonderer Anwendungsfall für die schmerzarme und definierte Injektion ist dabei die Injektion von Pharmakon in den Penis zum Zwecke seiner Stimulation.

Diese Aufgabe wird gemäß der Erfindung durch die Anwendung der Merkmale des Anspruchs 1 bzw. 13 gelöst. Die abhängigen Ansprüche 2 bis 12 bzw. 14 bis 17 geben zweckmäßige Weiterbildungen des Erfindungskonzepts an, wobei selbige teilweise gemeinsam, aber auch unabhängig voneinander zur Anwendung kommen können.

Die Erfindung, wie sie in den Ansprüchen gekennzeichnet ist, hat den Vorteil, daß der Gesamtvorgang der Injektion von Pharmaka praktisch selbsttätig abläuft, d.h., daß der Patient, außer dem Aufsetzen der Injektionseinrichtung auf das Organ bzw. den Körper lediglich noch den Vorgang starten muß. Ist die Injektionseinrichtung so gestaltet, daß die Kanülen in der Startstellung innerhalb des Gehäuses angeordnet sind und die Rückführung der Kanülen ebenfalls automatisch erfolgt, so kann bei geeigneter Wahl der Frequenz der gesamte Vorgang schmerzfrei und mit minimaler psychischer Beeinflussung ablaufen. Durch die Ausbildung der Injektionseinrichtung als Doppelspritze wird es möglich, daß sowohl Kanülenabstand als auch Einstichtiefe an die anatomischen Verhältnisse des jeweiligen Patienten vom Arzt optimal angepaßt werden können, was insbesondere bei dem bereits genannten speziellen Anwendungsfall von Bedeutung ist.

Durch Anwendung des erfindungsgemäßen Verfahrens sind auch vor allem die Entnahme von Körperflüssigkeiten sowie das Anlegen von Infusionskanülen schmerzarm möglich.

Die Erfindung wird nachfolgend anhand eines Ausführungsbeispiels und unter Bezugnahme auf die Zeichnungen näher erläutert. Es zeigen:

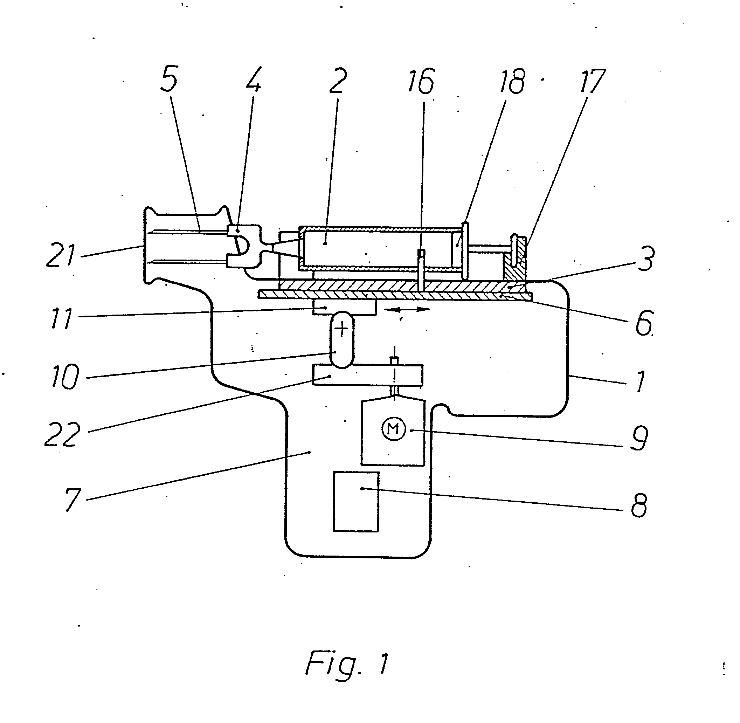

Fig. 1: Einen Schnitt durch die Injektionseinrichtung als Doppelspritze im Bereich der Vibrationseinrichtung mit Stromversorgungsteil,

Fig. 2: einen Schnitt durch die Injektionseinrichtung als Doppelspritze im Bereich des Spritzenantriebs.

In einem pistolenförmigen Gehäuse 1 ist im oberen Teil die Injektionsspritze 2 (eine Wegwerfspritze) in der Spritzenaufnahme 3 gehalten. Die Injektionsspritze 2 ist mit einem T-Stück 4 (ebenfalls ein Wegwerfteil) mit zwei Kanülen 5 bestückt. Die Spritzenaufnahme 3 ist auf einem Halter 6 befestigt, der auf seiner Unterseite mit einem rauhen Kupplungsbelag, z. B. Leder, belegt ist. Im Griff 7 des Gehäuses 1 ist die Stromversorgungseinrichtung, bestehend aus Batterien 8, Ladeeinrichtung und erforderlichen elektrischen Bauelementen, untergebracht. Weiterhin befindet sich im Griff ein Motor 9 mit einem Exzenter 22. Über ein Übertragungsglied 10 wird eine Schwingung mit einer Frequenz von ca. 50 Hz in Richtung der Achse der Injektionsspritze 2 (Pfeilrichtung) und mit einer Amplitude von ca. 1,5 mm erzeugt. Die Schwingung wird auf ein Kupplungsteil 11 übertragen, das mit seiner rauhen Oberfläche an dem Kupplungsbelag des Halters 6 anliegt und somit als Rutschkupplung wirkt. Der Motor 9 mit seinem Exzenter 22, dem Übertragungsglied 10 sowie dem Kupplungsteil 11 bilden zusammen eine Vibrationseinrichtung.

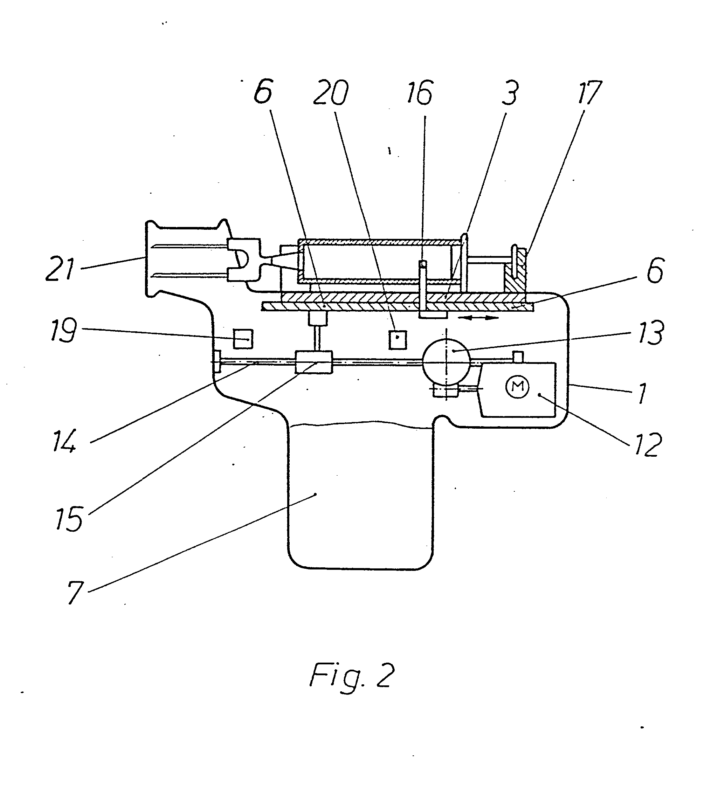

In Fig. 2 ist der Antrieb der Injektionsspritze 2 dargestellt. Ein Motor 12 treibt über ein Schneckengetriebe 13 eine Spindel 14, auf welcher ein Mitnehmer 15 in Längsrichtung bewegt wird, der am Anfang des Vorschubs den Halter 6 mit der eingespannten Injektionsspritze 2 bis zum Anschlag 16 bewegt, um den Einstich der Kanüle 5 in der vorgegebenen Tiefe in den Körper des Patienten vorzunehmen. Der Anschlag 16 ist einstellbar gestaltet, so daß mit ein und derselben Injektionsspritze 2 eine leichte Anpassung an den jeweiligen Anwendungsfall bzw. die gegebenen anatomischen Verhältnisse möglich ist. Der Abstand der Kanüle 5 kann bei Ausbildung der Injektionseinrichtung als Doppelspritze durch die Wahl eines geeigneten T-Stücks 4 ebenfalls an die anatomischen Bedingungen angepaßt werden.

Danach bewegt sich nur noch der in der Halterung 17 des Halters 6 gehaltene Teil des Kolbens 18 der Injektionsspritze 2. Der Kolben 18 bewirkt die Injektion der Pharmakonlösung, d.h. die Entleerung der Injektionsspritze 2, bis der Anschlag 19 mit seinem Endschalter erreicht ist. Durch Umschalten des Antriebs mittels eines im Griff eingebauten, nicht dargestellten Umschalters läuft der Mitnehmer 15 zurück, bis der Anschlag 20 erreicht ist, und zieht dadurch die Kanülen 5 aus dem Körper. Mittels des Anschlags 16 wird die Einstichtiefe im Bereich von 5 bis 10 mm eingestellt. Der gesamte Injektionsvorgang dauert ca. 90 s und läuft automatisch ab. Davon betragen die Einstichzeit ca. 15 s und die Entleerungszeit der Spritze (Injektion) ca. 60 s. Im Startzustand befinden sich die Spitzen der Kanülen 5 hinter einem Mundstück 21 des Gehäuses 1, welches auf die Haut des Patienten bzw. des Organs aufgesetzt wird. Durch die Ausführung des Motors 12 als drehzahlumschaltbaren Motor ist es möglich, die Einstichgeschwindigkeit der Kanülen 5 und die Injektionsgeschwindigkeit des Pharmakons unterschiedlich auszuführen und damit den optimalen Behandlungsbedingungen anzupassen. Ferner ist es möglich, bei Erreichen der größten Einstichtiefe bzw. nach Umpolung des Motors 12 zum Zwecke der Zurückführung der Kanülen in das Mundstück 21 den Motor 9 abzuschalten. Die Einrichtung ist elektrisch so ausgestattet, daß die Injetionseinrichtung im Gebrauch ohne Netzverbindung arbeitet.

Die im Ausführungsbeispiel detailliert dargestellte Doppelspritze mit ihren Einstellmöglichkeiten für Kanülenabstand und Einstichtiefe ist für den besonderen Fall der Injektion von Pharmakon in den Penis gestaltet. In diesem Anwendungsfall kommen als Pharmakon bevorzugt solche Präparate zum Einsatz, die zur Gruppe der arterienerweiternden und venenverengenden Medikamente gehören, wie z.B. Papaverin, Regitin und NaCl-Lösung im bestimmten Verhältnis zusammengesetzt. Unbeschadet dieses speziellen Anwendungsfalles ist die erfindungsgemäße Einrichtung vor allem für die Insulin-Injektion verwendbar, wobei sodann nur eine Kanüle auf die Injektionsspritze aufgesetzt ist.

**Ansprüche**

1. Injektionseinrichtung, die aus einem Antrieb für den axialen Vorschub und die Bewegung des Kolbens (18) einer Injektionsspritze (2) mit mindestens einer Kanüle (5) besteht, die auf einem beweglichen Halter (6) angeordnet ist, wobei der Antrieb Mittel zur Begrenzung des Vorschubs der Injektionsspritze (2) sowie der Bewegung des Kolbens (18) aufweist,
**dadurch gekennzeichnet, daß**
eine Vibrationseinrichtung (9, 10, 11, 22) für eine Frequenz von 40 bis 500 Hz vorgesehen ist, die mit dem Halter (6) derart vebunden ist, daß der auf dem Halter (6) angeordneten Injektionsspritze (2) während ihres Vorschubs und wahlweise auch während der Bewegung des Kolbens (18) eine Axialschwingung mit einer Amplitude von 0,1 bis 1,5 mm überlagert ist, daß der Antrieb für die Injektionsspritze (2) und den Kolben (18) sowie die verstellbar angeordneten Anschläge (18, 19, 20) so ausgebildet sind, daß der Einstich der Kanüle (5) stufig oder mit konstanter Geschwindigkeit einstellbar für eine Tiefe von 3 bis 15 mm in einer Zeit bis zu annähernd 20 s ausführbar ist.

2. Injektionseinrichtung nach Anspruch 1, dadurch gekennzeichnet, daß der Antrieb der Injektionsspritze (2) so ausgebildet ist, daß die Entleerung der Injektionsspritze (2) binnen einer Zeit von 10 bis 60 s erfolgt.

3. Injektionseinrichtung nach Anspruch 1 oder 2, dadurch gekennzeichnet, daß der Halter (6) der Injektionsspritze (2) mit der Vibrationseinrichtung (9, 10, 11, 22) mittels einer Kupplung, z. B. einer Rutschkupplung, im Eingriff steht.

4. Injektionseinrichtung nach Anspruch 1, 2 oder 3, dadurch gekennzeichnet, daß die Injektionsspritze (2) zur Verwendung als Doppelspritze mit einem auswechselbaren T-Stück (4) für zwei parallel verlaufende Kanülen (5) versehen ist.

5. Injektionseinrichtung nach Anspruch 4, dadurch gekennzeichnet, daß das T-Stück (4) mit konstantem oder variablem Abstand der Kanülen (5) ausgeführt und als Wegwerfeinheit gestaltet ist.

6. Injektionseinrichtung nach Anspruch 4 oder 5, dadurch gekennzeichnet, daß der Abstand der Kanülen (5) im T-Stück (4) 7 1/2, 11 oder 14 mm ist.

7. Injektionseinrichtung nach Anspruch 3, dadurch gekennzeichnet, daß bei Ausbildung der Kupplung als Rutschkupplung diese aus einer mit dem Halter (6) der Injektionsspritze (2) verbundenen, mit rauhem Kupplungsbelag versehenen Platte besteht, die durch Reibung mit einer rauhen Fläche, die auf dem Kupplungsteil (22) der Vibrationseinrichtung (9, 10, 11, 22) aufgebracht ist, verbunden ist.

8. Injektionseinrichtung nach Anspruch 1 bis 7, dadurch gekennzeichnet, daß der Antrieb für den Vorschub der Injektionsspritze (2) und für die Bewegung des Kolbens (18) in der Bewegungsrichtung umschaltbar ist.

9. Injektionseinrichtung nach Anspruch 1 bis 8, dadurch gekennzeichnet, daß als Antrieb für den Vorschub der Injektionsspritze (2) und für die Bewegung ihres Kolbens (18) sowie für die Vibrationseinrichtung (9, 10, 11, 22) ein einziger Motor vorgesehen ist.

10. Injektionseinrichtung nach Anspruch 1 bis 9, dadurch gekennzeichnet, daß die Mittel für die Begrenzung der Einstichtiefe der Kanüle (5) und der Bewegung des Kolbens (18) als Anschläge (16, 19, 20) ausgebildet und verstellbar sind.

11. Injektionseinrichtung nach Anspruch 1 bis 10, dadurch gekennzeichnet, daß die Mittel für die Begrenzung der Einstichtiefe der Kanüle (5) und der Bewegung des Kolbens (18) mit Endschaltern verbunden sind.

12. Injektionseinrichtung nach Anspruch 1 bis 11, dadurch gekennzeichnet, daß die Kanüle bzw. Kanülen (5) im Startzustand im Gehäuse (1) verdeckt angeordnet sind.

13. Verfahren zum Einführen einer oder mehrerer Kanülen in den menschlichen oder tierischen Körper
durch
Einstechen der Kanüle, die mit einer Druckerzeugungseinrichtung in Verbindung steht bzw. bringbar ist, in den Körper unter
-axialem Vorschub der Kanüle bis zu einer vorgegebenen Einstichtiefe
und
-gleichzeitiger und/oder anschließender Inbetriebnahme der Druckerzeugungseinrichtung,
dadurch gekennzeichnet, daß
die Kanüle während des Einstechens und gegebenenfalls auch noch danach während der Betätigung der Druckerzeugungseinrichtung in axiale Schwingungen im Frequenzbereich von 40 bis 500 Hz versetzt wird.

14. Verfahren nach Anspruch 13, dadurch gekennzeichnet, daß die Kanüle in axiale Schwingungen einer Amplitude von 0,1 bis 1,5 mm versetzt wird.

15. Verfahren nach Ansprüche 13 oder 14, dadurch gekennzeichnet, daß das Einstechen so durchgeführt wird, daß Einstichtiefen von 3 bis 15 mm in bis etwa 20 s erreicht werden.

16. Verfahren nach einem der Ansprüche 13 bis 15, dadurch gekennzeichnet, daß als Druckerzeugungseinrichtung eine Injektionsspritze verwendet und so betätigt wird, daß eine vorgegebene Flüssigkeitsmenge in 10 bis 60 s injiziert wird.

17. Verfahren nach einem der Ansprüche 13 bis 15, dadurch gekennzeichnet, daß als Druckerzeugungseinrichtung eine Peristaltikpumpe, ein Hebegefäß einer Infusionseinrichtung, das einen hydrostatischen Druck erzeugt, oder eine Absaugeinrichtung verwendet werden.

Fig. 1

Fig. 2